# EUROPEAN PATENT APPLICATION

(11) **EP 4 338 680 A1**
(43) Date of publication of application: **20.03.2024**
(21) Application number: 22201565.3
(22) Date of filing: 14.10.2022
(51) Int. Cl.: A61B 8/08

(54) **METHODS AND SYSTEMS FOR ANALYZING DIASTOLIC FUNCTION USING 2D ECHOCARDIOGRAPHIC IMAGES**

(30) Priority: 16.09.2022 US 202263407288 P
(71) Applicant: Koninklijke Philips N.V., 5656 AG Eindhoven (NL)
(72) Inventor: SADEGHI, Seyedali, Eindhoven (NL); OLIVEIRA, Lucas, Eindhoven (NL); GESSERT, Nils Thorben, 5656AG Eindhoven (NL); ESLAMI, Parastou, 5656AG Eindhoven (NL); WEHLE, Simon, Eindhoven (NL); WAECHTER-STEHLE, Irina, Eindhoven (NL); PRABHU, David, Eindhoven (NL)
(74) Representative: Philips Intellectual Property & Standards

(57) **Abstract**

A method (100) for classifying a patient's diastolic function, comprising: (i) receiving (120), from an ultrasound device (280), a plurality of 2D echocardiographic images of the patient's heart; (ii) analyzing (150), by a trained diastolic function prediction algorithm, the plurality of 2D echocardiographic images of the patient's heart to estimate left ventricular end-diastolic pressure (LVEDP); (iii) classifying (160) the patient's diastolic function as normal or abnormal based on the estimated LVEDP; and (iv) providing (170), to a user via a user interface, an indication of the patient's diastolic function as normal or abnormal.

## Description

### FIELD OF THE INVENTION

The present disclosure is directed generally to methods and systems for classifying a subject's diastolic function.

### BACKGROUND OF THE INVENTION

Heart failure, which can be defined as the inability of the heart to provide sufficient cardiac output while maintaining normal filling pressures, affects at least 26 million people worldwide and is estimated to increase by 46% by 2030. Two types of heart failure exist: (1) heart failure with reduced ejection fraction (HFrEF) and (2) heart failure with preserved ejection fraction (HFpEF). The latter, HFpEF, makes up 50% of heart failure cases and is characterized by impaired relaxation of the left ventricle (LV) during diastole (diastolic dysfunction) and increased filling pressures caused by altered LV mechanical properties, most notably higher stiffness. Conditions such as cardiac amyloidosis, coronary artery disease, valvular disease, hypertrophic cardiomyopathy (HCM), pericardial disease, and hypertension can produce HFpEF.

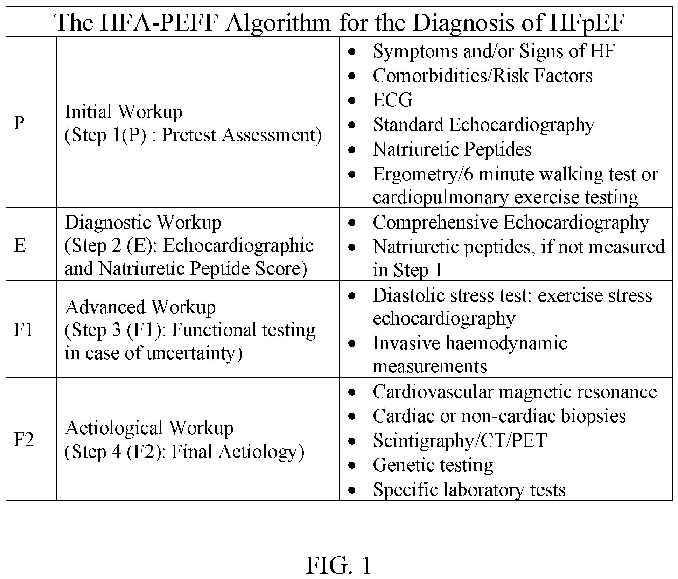

While there are current guidelines for the diagnosis of diastolic dysfunction (including history, physical examination, and echocardiography, and further including cardiac catheterization if necessary), these guidelines are complicated and rarely followed. As a result, definitively diagnosing diastolic dysfunction is challenging. Conditions such as cardiac amyloidosis, coronary artery disease (CAD), valvular disease, hypertrophic cardiomyopathy (HCM), pericardial disease, and hypertension can produce diastolic dysfunction. 2D Echocardiography is the primary imaging modality for diastolic dysfunction. However, it cannot be used alone for a definite diagnosis. An illustration of existing diagnostic workup stages for definite and differential diagnosis of HFpEF is shown in FIG. 1, identifying the full complexity of differential diagnosis workflow. As can be seen, ultrasound is not used in step F2, the differential diagnosis step of the process.

Currently, non-invasive assessment of LVEDP using echocardiography relies on complex guidelines and are rarely followed. A sizeable number of patients determined to have diastolic dysfunction using a non-invasive assessment are ultimately found to have normal LVEDP, and vice-versa.

For borderline cases regarding a diastolic dysfunction diagnosis, deciding whether to stress test, catheterize, or perform advanced imaging such as positron emission tomography (PET) or cardiovascular magnetic resonance imaging (CMR) is a subjective assessment of the cardiologist. This can lead to inefficient use of resources and suboptimal patient care. There is agreement in the cardiac imaging community that there is a strong need for standardization, guided by clinically tailored health technology assessment studies, to obtain an appropriate utilization of the different available tools with greater efficiency and efficacy of care and better patient outcomes. However, currently, there is no wellaccepted or intelligent patient-specific method for predicting diastolic dysfunction, particularly using 2D ultrasound B-mode images alone. Therefore, there is a crucial need for establishing an intelligent data-driven decision support tool for analyzing diastolic function and definitely diagnosing diastolic dysfunction.

### SUMMARY OF THE INVENTION

Accordingly, there is a continued need for methods and systems for more accurate and efficient analysis of diastolic function. Various embodiments and implementations herein are directed to a method and system configured to classify a patient's diastolic function. A system, such as for example a diastolic function analysis system, receives, from an ultrasound device, a plurality of 2D echocardiographic images of the patient's heart. A trained diastolic function prediction algorithm analyzes the plurality of 2D echocardiographic images of the patient's heart to estimate left ventricular end-diastolic pressure (LVEDP). The system uses the estimated LVEDP to classify the patient's diastolic function as normal or abnormal, and optionally classifies the patient's diastolic function as intermediate or indeterminate. The system then provides an indication of the patient's classified diastolic function to a user via a user interface.

Generally, in one aspect, a method for classifying a patient's diastolic function is provided. The method includes: (i) receiving, from an ultrasound device, a plurality of 2D echocardiographic images of the patient's heart; (ii) analyzing, by a trained diastolic function prediction algorithm, the plurality of 2D echocardiographic images of the patient's heart to estimate left ventricular end-diastolic pressure (LVEDP); (iii) classifying the patient's diastolic function as normal or abnormal based on the estimated LVEDP; and (iv) providing, to a user via a user interface, an indication of the patient's diastolic function as normal or abnormal.

According to an embodiment, the method further includes the step of selecting, by a trained image selection algorithm, a subset of the plurality of received 2D echocardiographic images of the patient's heart for analysis, wherein the image selection algorithm is trained to select 2D echocardiographic images as being optimal for analysis, wherein said analyzing step comprises analyzing the selected subset of the plurality of received 2D echocardiographic images of the patient's heart.

According to an embodiment, the method further includes the step of receiving clinical information about the subject, wherein the trained diastolic dysfunction prediction algorithm also analyzes the received clinical information to classify the patient's diastolic function as normal or abnormal.

According to an embodiment, the method further includes the step of receiving, via a user interface, input from a user to initiate an analysis by the trained diastolic dysfunction prediction algorithm.

According to an embodiment, the patient's diastolic function is classified as normal when the estimated LVEDP is equal to or less than 10 mmHg. According to an embodiment, the patient's diastolic function is classified as abnormal when the estimated LVEDP is equal to or greater than 15 mmHg. According to an embodiment, the trained diastolic function prediction algorithm is further configured to classify the patient's diastolic function as indeterminate when the estimated LVEDP is between 10 mmHg and 15 mmHg.

According to another aspect is a non-transitory computer-readable storage medium comprising computer program code instructions which, when executed by a processor, enables the processor to carry out the method of: (i) receiving, from an ultrasound device, a plurality of 2D echocardiographic images of the patient's heart; (ii) analyzing, by a trained diastolic function prediction algorithm, the plurality of 2D echocardiographic images of the patient's heart to estimate left ventricular end-diastolic pressure (LVEDP); (iii) classifying the patient's diastolic function as normal or abnormal based on the estimated LVEDP; and (iv) providing, to a user via a user interface, an indication of the patient's diastolic function as normal or abnormal.

According to another aspect is a method for classifying a patient's diastolic function. The method includes: (i) receiving, from an ultrasound device, a plurality of 2D echocardiographic images of the patient's heart; (ii) selecting, by a trained image selection algorithm, a subset of the plurality of received 2D echocardiographic images of the patient's heart for analysis, wherein the image selection algorithm is trained to select 2D echocardiographic images as being optimal for analysis; (iii) receiving clinical information about the subject, wherein the clinical information comprises demographic information; (iv) analyzing, by a trained diastolic function prediction algorithm, the selected subset of 2D echocardiographic images of the patient's heart and the received clinical information to estimate left ventricular end-diastolic pressure (LVEDP); (v) classifying the patient's diastolic function as normal, indeterminate, or abnormal based on the estimated LVEDP, wherein the patient's diastolic function is classified as normal when the estimated LVEDP is equal to or less than 10 mmHg, wherein the patient's diastolic function is classified as abnormal when the estimated LVEDP is equal to or greater than 15 mmHg, and wherein the trained diastolic dysfunction prediction algorithm is further configured to classify the patient's diastolic function as indeterminate when the estimated LVEDP is between 10 mmHg and 15 mmHg; and (vi) providing, to a user via a user interface, an indication of the patient's diastolic function as normal, indeterminate, or abnormal.

According to an aspect is a system for classifying a patient's diastolic function. The system includes: an ultrasound device configured to obtain a plurality of 2D echocardiographic images of the patient's heart; a trained diastolic function prediction algorithm trained to estimate left ventricular end-diastolic pressure (LVEDP) from the plurality of 2D echocardiographic images of the patient's heart; a user interface; and a processor configured to: (i) analyze, using the trained diastolic function prediction algorithm, the plurality of 2D echocardiographic images of the patient's heart to estimate LVEDP; (ii) classify the patient's diastolic function as normal or abnormal based on the estimated LVEDP; and (iii) direct the user interface to provide an indication of the patient's diastolic function as normal or abnormal.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

### BRIEF DESCRIPTION OF THE DRAWINGS

In the drawings, like reference characters generally refer to the same parts throughout the different views. The figures showing features and ways of implementing various embodiments and are not to be construed as being limiting to other possible embodiments falling within the scope of the attached claims. Also, the drawings are not necessarily to scale, emphasis instead generally being placed upon illustrating the principles of the various embodiments.
Fig. 1 is a chart of diagnostic workup stages for definite and differential diagnosis of HFpEF, according to the prior art.
Fig. 2 is a flowchart of a method for classifying a patient's diastolic function, in accordance with an embodiment.
Fig. 3 is a schematic representation of a diastolic function analysis system, in accordance with an embodiment.
Fig. 4 is a flowchart of a method for training a diastolic function prediction algorithm, in accordance with an embodiment.
Fig. 5A is a graph of diastolic function prediction evaluation, in accordance with an embodiment.
Fig. 5B is a table of diastolic function prediction evaluation, in accordance with an embodiment.
Fig. 6 is a schematic representation of the presentation of a diastolic function analysis, in accordance with an embodiment.
Fig. 7 is a flowchart of a method for classifying a patient's diastolic function, in accordance with an embodiment.

### DETAILED DESCRIPTION OF EMBODIMENTS

The present disclosure describes various embodiments of a system and method configured to generate and present a classification of a subject's diastolic function. More generally, Applicant has recognized and appreciated that it would be beneficial to provide an intelligent data-driven decision support tool for diastolic function. Accordingly, a diastolic function analysis system receives, from an ultrasound device, a plurality of 2D echocardiographic images of the patient's heart. A trained diastolic function prediction algorithm analyzes the plurality of 2D echocardiographic images of the patient's heart to estimate left ventricular end-diastolic pressure (LVEDP). The system uses the estimated LVEDP to classify the patient's diastolic function as normal or abnormal, and optionally classifies the patient's diastolic function as intermediate or indeterminate. The system then provides an indication of the patient's classified diastolic function to a user via a user interface. The provided indication of the patient's classified diastolic function can then be utilized by a healthcare professional to implement a healthcare treatment for the subject.

According to an embodiment is a deep-learning (DL) algorithm based solely on analysis of 2D echocardiographic images to accurately identify patients with elevated LV filling pressures. All existing models for diastolic function assessment using machine-learning require ultrasound parameters - that is, ultrasound measurement values - to perform an analysis. There is no deep-learning-based solution using only images as input for the classification of diastolic dysfunction. One advantage of such a deep-learning-based model is that it can benefit institutions that do not have access to premium ultrasound systems (for Doppler measurements, tissue Doppler, etc.) if the deep learning model requires only B-mode ultrasound images as input. In addition, ultrasound images may provide better accuracy compared to the ultrasound values alone as input. The successful 2D echocardiographic classification of elevated LVEDP improves upon the existing paradigm for noninvasive diastolic function assessment, allowing for improved detection and treatment of heart failure.

According to an embodiment, the systems and methods described or otherwise envisioned herein can, in some non-limiting embodiments, be implemented as an element for a commercial product for ultrasound imaging or analysis, or as an element for a commercial product for cardiovascular analysis such as Philips^{®} IntelliSpace Cardiovascular (ISCV) (available from Koninklijke Philips NV, the Netherlands), or as an element for a commercial product for patient analysis or monitoring, such as the Philips Patient Flow Capacity Suite (PFCS), or any suitable system.

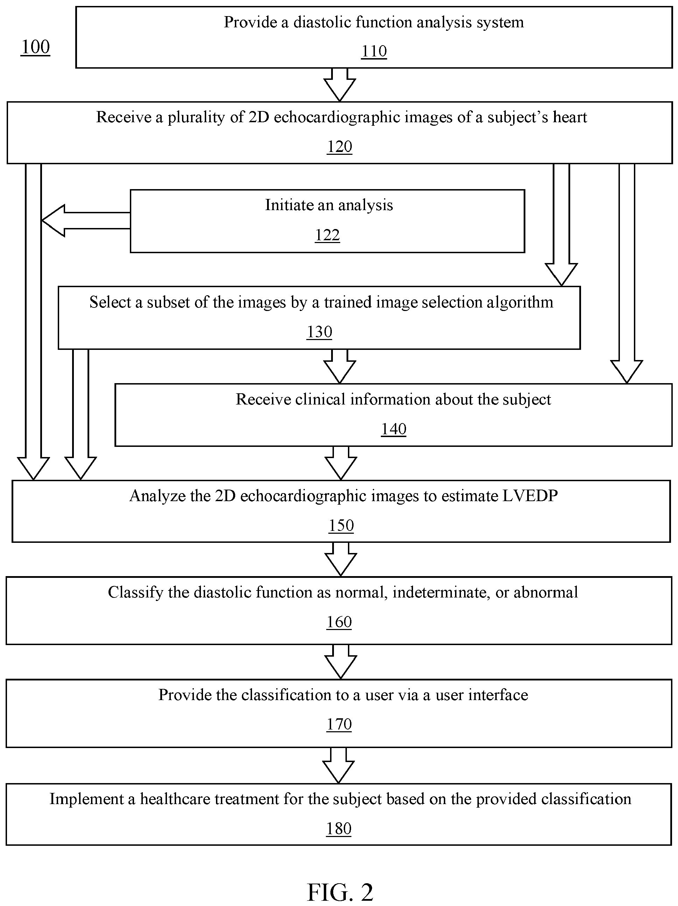

Referring to Fig. 2, in one embodiment, is a flowchart of a method 100 for classifying a patient's diastolic function using a diastolic function analysis system 200. The methods described in connection with the figures are provided as examples only, and shall be understood not limit the scope of the disclosure. The diastolic function analysis system can be any of the systems described or otherwise envisioned herein. The diastolic function analysis system can be a single system or multiple different systems.

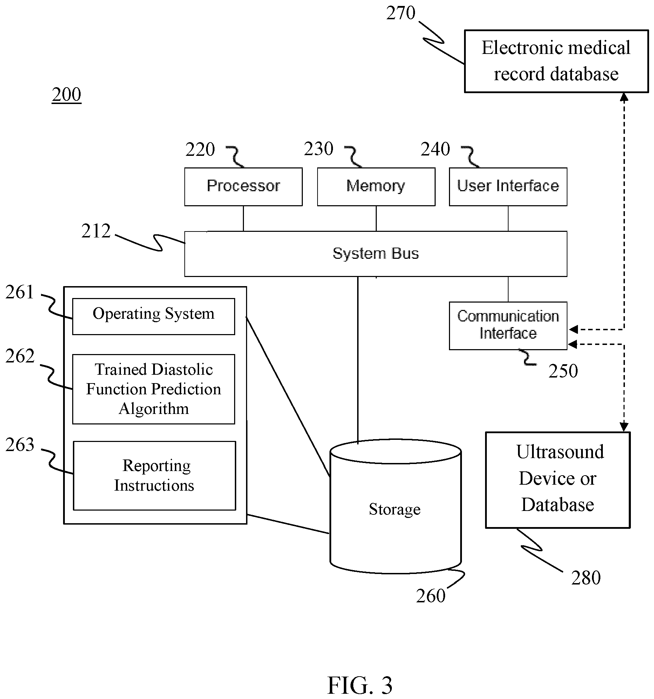

At step 110 of the method, a diastolic function analysis system 200 is provided. Referring to an embodiment of a diastolic function analysis system 200 as depicted in Fig. 3, for example, the system comprises one or more of a processor 220, memory 230, user interface 240, communications interface 250, and storage 260, interconnected via one or more system buses 212. It will be understood that Fig. 3 constitutes, in some respects, an abstraction and that the actual organization of the components of the system 200 may be different and more complex than illustrated. Additionally, diastolic function analysis system 200 can be any of the systems described or otherwise envisioned herein. Other elements and components of diastolic function analysis system 200 are disclosed and/or envisioned elsewhere herein.

At step 120 of the method, the diastolic function analysis system receives a plurality of 2D echocardiographic images of a subject's heart. The subject can be a patient, user, or any other individual for which an analysis is being performed. The plurality of 2D echocardiographic images of the subject's heart can be received as a result of an ultrasound analysis of the subject's heart. The ultrasound analysis of the subject's heart can be any analysis sufficient to provide ultrasound data about the subject that can be utilized in downstream steps of the method. The ultrasound analysis of the subject's heart can be obtained using any ultrasound methodology or device capable of providing the ultrasound data utilized in downstream steps of the method. According to an embodiment, the ultrasound analysis of the subject's heart comprises a plurality of images obtained by an ultrasound device.

According to an embodiment, diastolic function analysis system 200 comprises an ultrasound device capable of acquiring the required ultrasound images or analysis. According to another embodiment, diastolic function analysis system 200 is in wired and/or wireless communication with a local or remote ultrasound device capable of acquiring the required ultrasound images or analysis. According to another embodiment, diastolic function analysis system 200 is in wired and/or wireless communication with a local or remote database which stores the ultrasound images or analysis. The diastolic function analysis system 200 can obtain the required ultrasound images or analysis from one or more of these sources.

According to an embodiment, the ultrasound analysis of the subject's heart is obtained by an ultrasound imaging specialist as part of a routine analysis of the subject or in response to possible or known medical issues being experienced by the subject. The ultrasound analysis may be performed or obtained for immediate or near-term analysis by the methods and systems described or otherwise envisioned herein, or may be performed or obtained for a future analysis by the methods and systems described or otherwise envisioned herein. According to an embodiment, the ultrasound analysis of the subject's heart comprises 2D images or recording, 3D images or recording from which 2D imagery can be extracted, and/or both.

At optional step 122 of the method, a user of the diastolic function analysis system 200 may initiate an analysis of 2D echocardiographic images of a patient by the trained image selection algorithm. For example, an analysis may be performed while images are being obtained or after images are obtained. After images are obtained may be immediately or soon after an examination of the patient is performed, or an analysis may be made using stored images some period of time after an examination is performed. The request or command to initiate the analysis of 2D echocardiographic images of the patient is submitted to the diastolic function analysis system via a user interface. The user interface can be any device or system that allows information to be conveyed and/or received, and may include a display, a mouse, and/or a keyboard for receiving user commands. The user interface can be a component of the diastolic function analysis system, and/or the request or command to initiate the analysis may be communicated by wired and/or wireless communication to the system from another device or system.

At optional step 130 of the method, a trained image selection algorithm of the diastolic function analysis system 200 analyzes the received plurality of 2D echocardiographic images and identifies a subset of the images as being optimal for further analysis. The subset of the images may be all or less than all of the received plurality of 2D images. According to an embodiment, the image selection algorithm is trained to select an image as optimal for further analysis based on any of a plurality of different features. In the context of the present disclosure, an optimal image (or an image being identified as being optimal) out of a plurality of images preferably refers to the fact that said image is better suited for further analysis than the other image(s) of said plurality. For example, optimal may be defined as comprising detectable features in an image, useable by the trained diastolic function algorithm. Optimal may comprise aspects such as clarity of the image, ensuring that the image or structures or features in the image are not blurry, that structures or features are present and/or visible, and other aspects.

Once the trained image selection algorithm selects the subset of the plurality of received 2D echocardiographic images, the subset can be utilized immediately or may be stored in local or remote storage for use in further steps of the method.

At optional step 140 of the method, the diastolic function analysis system receives clinical information about the patient. The clinical information about the subject can be any information relevant to or useful in any downstream step of the method, including as input to the trained diastolic function prediction algorithm to estimate the patient's left ventricular end-diastolic pressure (LVEDP). According to an embodiment, the clinical information about the subject comprises one or more of an ultrasound exam type, a reason for the ultrasound analysis, age of the subject, sex of the subject, body mass index of the subject, disease status or diagnosis, medical treatment, and medical diagnosis, among many other types of clinical information. For example, age may affect the interpretation of diastolic function. Therefore, these clinical information data can be significant factors affecting LVEDP. Accordingly, the received information can be any information relevant to a patient analysis as described or otherwise envisioned herein.

The diastolic function analysis system can receive patient clinical information from a variety of different sources. According to an embodiment, the diastolic function analysis system is in communication with an electronic medical records database from which the patient clinical information may be obtained or received. According to an embodiment, the diastolic function analysis system comprises an electronic medical record database or system 270 which is optionally in direct and/or indirect communication with system 200. According to another embodiment, the diastolic function analysis system may obtain or receive the information from equipment or a healthcare professional obtaining that information directly from the patient.

The patient clinical information received by the diastolic function analysis system may be processed by the system according to methods for data handling and processing/preparation, including but not limited to the methods described or otherwise envisioned herein. The patient clinical information received by the diastolic function analysis system may be utilized, before or after processing, immediately or may be stored in local or remote storage for use in further steps of the method.

At step 150 of the method, a trained diastolic function prediction algorithm analyzes the received plurality of 2D echocardiographic images of the patient's heart, or the selected subset of the plurality of 2D echocardiographic images of the patient's heart, to estimate the left ventricular end-diastolic pressure (LVEDP) of the patient's heart. According to one embodiment, the trained diastolic function prediction algorithm may also analyze received clinical information about the patient, but this is not essential. Other input to the trained diastolic function prediction algorithm is possible.

According to an embodiment, the trained diastolic function prediction algorithm estimates the LVEDP of the patient's heart using a wide variety of different classifier and/or machine learning algorithms as described or otherwise envisioned herein. According to an embodiment, the trained diastolic function prediction algorithm of the diastolic function analysis system can be trained according to a wide variety of methods and approaches. As one example, the algorithm may comprise a neural network approach.

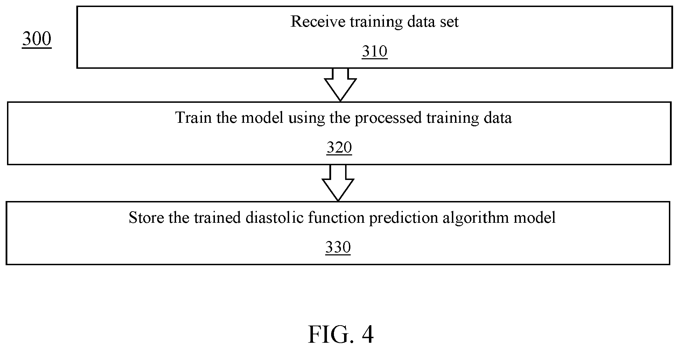

Referring to Fig. 4, in one embodiment, is a flowchart of a method 300 for training the diastolic function prediction algorithm of the diastolic function analysis system. At step 310 of the method, the system receives a training data set comprising training data about a plurality of patients, such as historical patient data. The training data can comprise, for example, input for previous or historical patients for which data about diastolic function was obtained. For example, the training data can comprise information about patients that underwent 2D echocardiography and left heart catheterization with LVEDP measurement, with a variety of different results. Accordingly, the diastolic function prediction algorithm can be trained to predict LVEDP based on 2D echocardiography. The training data may further comprise demographic information about the previous or historical patients, and that training data may be utilized to train the diastolic function prediction algorithm as well. The training data may be stored in and/or received from one or more databases. The database may be a local and/or remote database. For example, the diastolic function analysis system may comprise a database of training data.

According to an embodiment, the diastolic function analysis system may comprise a data pre-processor or similar component or algorithm configured to process the received training data. For example, the data pre-processor analyzes the training data to remove noise, bias, errors, and other potential issues. The data pre-processor may also analyze the input data to remove low-quality data. Many other forms of data pre-processing or data point identification and/or extraction are possible.

At step 320 of the method, the system trains the machine learning algorithm, which will be the algorithm utilized in analyzing the input information as described or otherwise envisioned. The machine learning algorithm is trained using the training data set according to known methods for training a machine learning algorithm. According to an embodiment, the algorithm is trained, using the processed training dataset, to analyze a plurality of 2D echocardiographic images of a patient's heart to estimate the left ventricular end-diastolic pressure (LVEDP) of the patient's heart, and thereby classify the patient's diastolic function as normal, indeterminate, or abnormal based on the estimated LVEDP.

At step 330 of the method, the trained diastolic function prediction algorithm of the diastolic function analysis system is stored for future use. According to an embodiment, the model may be stored in local or remote storage.

According to an embodiment, ground truth for LVEDP can be collected from the definite results of diagnostic tests for a plurality of patients, combining 2D echocardiography and left heart catheterization with LVEDP measurement. For example, ground truth for diastolic function status can be collected from the invasive catheterization for LVEDP measurement, which can optionally be analyzed by an expert panel of cardiologists for some or all of the patients. Based on the aforementioned input and ground truth on the diastolic function, a deep-learning-based algorithmic model can be implemented. According to an embodiment, this supervised learning approach could be an institution-agnostic tool for diastolic function classification tasks. The accuracy of an AI-based learning network can get stronger over time by adding more data to it, such as a self-learning algorithm.

### EXAMPLE 1 - TRAINING DATA

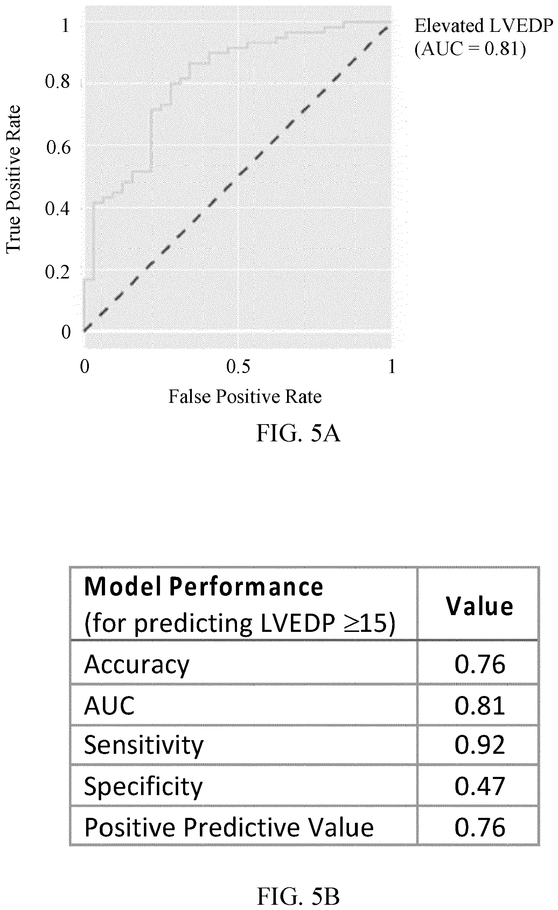

According to one non-limiting example, training data comprised a database containing 10,000 Digital Imaging and Communications in Medicine (DICOMs) from 632 studies of patients who underwent 2D echocardiography and left heart catheterization with LVEDP measurement on the same day at the University of Chicago Medical center in a retrospective study. Images were divided randomly into training (80%) and test (20%) datasets. A trained DL algorithm (convolutional neural network (CNN)) was developed using the training dataset using 2-D, apical 4-chamber images (n=5,212 from 626 studies) to automatically classify AI-derived LVEDP into two categories: elevated or abnormal (LVEDP ≥15 mmHg) and normal (LVEDP ≤10 mmHg) as validated by true invasive LVEDP. Notably, other ranges could have been utilized, including using 10-15 mmHg as being indeterminate. In the training set, the automated DL showed high levels of diagnostic accuracy reflected by a high AUC value of 1.00, and overall accuracy of 1.00. In the test dataset, the DL model-based calculation performance remained strong (AUC 0.81, accuracy 0.76), as shown in Figs. 5A and 5B. According to an embodiment, the training data may also incorporate of Doppler images and/or measurements.

Returning to method 100 in Fig. 2, at step 160 the diastolic function analysis system, with the diastolic function prediction algorithm or another algorithm, utilizes the estimated left ventricular end-diastolic pressure (LVEDP) of the patient's heart (determined in step 150 by the trained diastolic function prediction algorithm) to classify the patient's diastolic function. Classification based on the LVEDP can be determined by comparing the estimated LVEDP to predetermined or learned ranges or thresholds, or by other methods.

For example, according to one embodiment, a patient's diastolic function is classified as normal when the estimated LVEDP is equal to or less than 10 mmHg. According to an embodiment, the patient's diastolic function is classified as abnormal when the estimated LVEDP is equal to or greater than 15 mmHg. According to an embodiment, the patient's diastolic function is classified as indeterminate when the estimated LVEDP is between 10 mmHg and 15 mmHg. Although these thresholds are provided as examples, other thresholds are possible. As described or otherwise envisioned herein, the thresholds can be predetermined, or they can be learned during the initial or subsequent training of the diastolic function prediction algorithm.

At step 170 of the method, the classification of the patient's diastolic function - which may be normal, indeterminate, abnormal, or other possible classifications - is provided to a user via a user interface of the diastolic function analysis system 200. The indication of the patient's diastolic function classification can be communicated via text or via a visualization. For example, the indication can be communicated via a simple textual indication such as "NORMAL," "ABNORMAL," "INDETERMINATE," and so on. The indication may include additional information such as information about the patient, including the optional demographic information provided to and/or used by the system, the patient's name, and/or a treatment or intervention recommendation, among many other types of information. The indication may also include the estimated LVEDP determined by the diastolic function prediction algorithm, such as "8 mmHg," "13 mmHg," and "18 mmHg," as a couple of non-limiting examples.

According to an embodiment, the information may be communicated by wired and/or wireless communication to a user interface and/or to another device. For example, the system may communicate the information to a mobile phone, computer, laptop, wearable device, and/or any other device configured to allow display and/or other communication of the report. The user interface can be any device or system that allows information to be conveyed and/or received, and may include a display, a mouse, and/or a keyboard for receiving user commands.

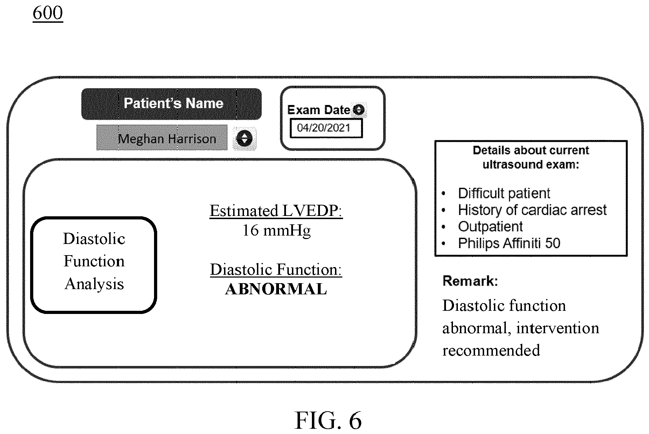

According to an embodiment, the provided classification may be communicated by a visualization such as a dashboard or other possible visualization. For example, referring to Fig. 6, in one embodiment, is a schematic representation of a possible visualization of the classification of the patient's diastolic function. The dashboard provides information including the patient's name, the exam date, and details about the current ultrasound exam. The dashboard also provides information about the Diastolic Function Analysis performed by the diastolic function prediction algorithm, including an estimated LVEDP (which is 16 mmHg in this example), and the diastolic function classification (which is ABNORMAL in this example). The dashboard also provides a recommendation for treatment or intervention or follow-up (which is "intervention recommended" in this example, although more specific treatment or intervention information may be possible).

According to an embodiment of a diastolic function analysis system 200, the system can comprise a user interface to facilitate the methods described or otherwise envisioned herein. Accordingly, the user interface can comprise a "decision support tool for diastolic function" button or activator that appears on the ultrasound scanner touch panel or a workspace such as a Philips Intellispace Cardiovascular (ISCV) platform for the user to launch the application.

At optional step 180 of method 100 depicted in Fig. 2, the provided indication of the patient's estimated LVEDP and/or classification of diastolic function can be utilized by a healthcare professional to implement a healthcare treatment for the subject. For example, a clinician or other decisionmaker utilizes the patient's estimated LVEDP and/or classification of diastolic function for patient care decision-making. For example, the healthcare recommendation may comprise a recommendation to initiate, continue, or stop a particular treatment configured to address abnormal diastolic function, which is based on the determined estimated LVEDP and/or the classification of diastolic function. Implementation can comprise a prescription, order, additional testing, and/or another implementation. Many other implementations are possible.

According to an embodiment, the diastolic function analysis system 200 provides a userfriendly and intelligent patient-specific decision support tool for diastolic function analysis. There is currently no intelligent non-invasive decision tool for diastolic dysfunction determination for cardiologists. Subjective decisions about the need for advanced follow-up invasive catheterization and stress exam lead to misdiagnosis, increased cost, inefficient use of resources, and suboptimal patient care. The diastolic function analysis system provides an intelligent patient-specific deep learning-based tool to inform cardiologists about the presence of diastolic dysfunction by leveraging 2D echocardiographic images of the left ventricle. Therefore, this system empowers clinicians with a decision support tool for diastolic dysfunction diagnosis so physicians can better plan for a patient's care. The system utilizes information retrieved and/or calculated from 2D ultrasound images from a subject's left ventricle (LV) in an exam, where the ultrasound images can be established in routine 2D image acquisition from the LV of the heart. According to one possible embodiment, the preferred 2D image is taken with the apical 4-chamber view, with no LV foreshortening, and good image quality.

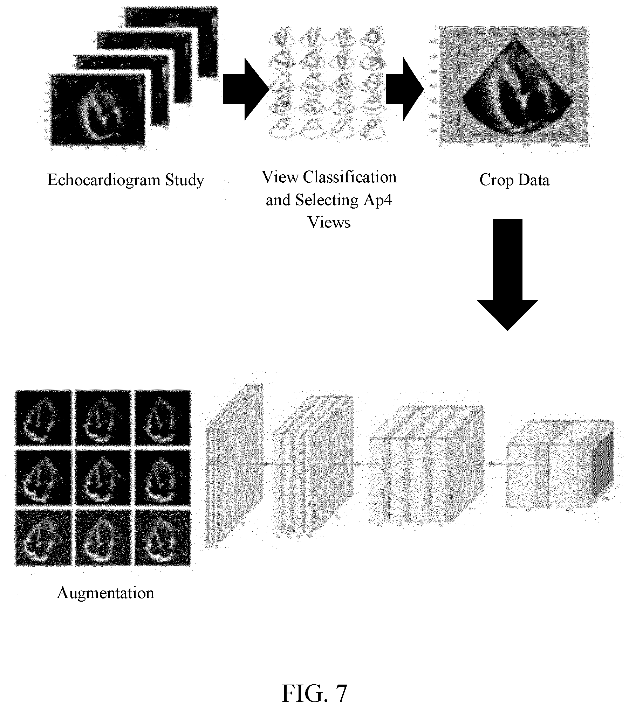

Referring to Fig. 7, in one embodiment, is a schematic representation showing the input and output of the AI-based network for estimating diastolic dysfunction. The model input is the echocardiographic images (or sequences of stacked images per study/per patient) and the output is the binary classification of diastolic function status (normal, indeterminate, abnormal). The ground truth for the diastolic function status is collected from the invasive catheterization for LVEDP measurement, which is analyzed by an expert panel of cardiologists for each patient. Based on the aforementioned input and ground truth on the diastolic function, a deep-learning-based algorithmic model can be implemented.

According to an embodiment, the diastolic function analysis system may utilize additional input to the system and/or to the trained diastolic function prediction algorithm. In addition to the ultrasound images, additional input may be considered for improving the deep learning network. These additional inputs include but are not limited to the patient's demographic (age, gender, BMI, history of diastolic dysfunction), other ultrasound biomarkers: comprehensive ultrasound exams need several quantitative parameters to be collected (such as global longitudinal strain (GLS), ejection fraction, left atrium volume index (LAVI), mitral propagation velocity (Vp), mitral inflow at the beginning of diastolic phase (E), tissue velocity at the beginning of diastolic phase (e'), tricuspid regurgitation (TR) velocity, relative wall thickness (RWT), LV thickness, septum, valves, and RV thickness.

According to an embodiment, the diastolic function analysis system may also provide, along with the classification of the patient's diastolic function and/or the estimated LVEDP determined by the diastolic function prediction algorithm, a saliency map. For example, saliency maps can show the area(s) in the input image that the model was particularly focused on.

Referring to Fig. 3 is a schematic representation of a diastolic function analysis system 200. System 200 may be any of the systems described or otherwise envisioned herein, and may comprise any of the components described or otherwise envisioned herein. It will be understood that Fig. 3 constitutes, in some respects, an abstraction and that the actual organization of the components of the system 200 may be different and more complex than illustrated.

According to an embodiment, system 200 comprises a processor 220 capable of executing instructions stored in memory 230 or storage 260 or otherwise processing data to, for example, perform one or more steps of the method. Processor 220 may be formed of one or multiple modules. Processor 220 may take any suitable form, including but not limited to a microprocessor, microcontroller, multiple microcontrollers, circuitry, field programmable gate array (FPGA), application-specific integrated circuit (ASIC), a single processor, or plural processors.

Memory 230 can take any suitable form, including a non-volatile memory and/or RAM. The memory 230 may include various memories such as, for example L1, L2, or L3 cache or system memory. As such, the memory 230 may include static random access memory (SRAM), dynamic RAM (DRAM), flash memory, read-only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 200. It will be apparent that, in embodiments where the processor implements one or more of the functions described herein in hardware, the software described as corresponding to such functionality in other embodiments may be omitted.

User interface 240 may include one or more devices for enabling communication with a user. The user interface can be any device or system that allows information to be conveyed and/or received, and may include a display, a mouse, and/or a keyboard for receiving user commands. In some embodiments, user interface 240 may include a command line interface or graphical user interface that may be presented to a remote terminal via communication interface 250. The user interface may be located with one or more other components of the system, or may located remote from the system and in communication via a wired and/or wireless communications network.

Communication interface 250 may include one or more devices for enabling communication with other hardware devices. For example, communication interface 250 may include a network interface card (NIC) configured to communicate according to the Ethernet protocol. Additionally, communication interface 250 may implement a TCP/IP stack for communication according to the TCP/IP protocols. Various alternative or additional hardware or configurations for communication interface 250 will be apparent.

Storage 260 may include one or more machine-readable storage media such as read-only memory (ROM), random-access memory (RAM), magnetic disk storage media, optical storage media, flash-memory devices, or similar storage media. In various embodiments, storage 260 may store instructions for execution by processor 220 or data upon which processor 220 may operate. For example, storage 260 may store an operating system 261 for controlling various operations of system 200.

It will be apparent that various information described as stored in storage 260 may be additionally or alternatively stored in memory 230. In this respect, memory 230 may also be considered to constitute a storage device and storage 260 may be considered a memory. Various other arrangements will be apparent. Further, memory 230 and storage 260 may both be considered to be non-transitory machine-readable media. As used herein, the term non-transitory will be understood to exclude transitory signals but to include all forms of storage, including both volatile and non-volatile memories.

While system 200 is shown as including one of each described component, the various components may be duplicated in various embodiments. For example, processor 220 may include multiple microprocessors that are configured to independently execute the methods described herein or are configured to perform steps or subroutines of the methods described herein such that the multiple processors cooperate to achieve the functionality described herein. Further, where one or more components of system 200 is implemented in a cloud computing system, the various hardware components may belong to separate physical systems. For example, processor 220 may include a first processor in a first server and a second processor in a second server. Many other variations and configurations are possible.

According to an embodiment, the electronic medical record database 270 is an electronic medical records database from which the information about a patient, including clinical information, may be obtained or received. The electronic medical record database 270 can also be the database from which training data is obtained or received. The electronic medical records database may be a local or remote database and is in direct and/or indirect communication with the diastolic function analysis system 200. Thus, according to an embodiment, the diastolic function analysis system comprises an electronic medical record database or system 270.

According to an embodiment, the system comprises one or more ultrasound devices 280 capable of acquiring the required ultrasound images or analysis. According to another embodiment, diastolic function analysis system 200 is in wired and/or wireless communication with a local or remote ultrasound device 280 capable of acquiring the required ultrasound images or analysis. According to another embodiment, diastolic function analysis system 200 is in wired and/or wireless communication with a local or remote database 280 which stores the ultrasound images or analysis. The diastolic function analysis system 200 can obtain the required ultrasound images or analysis from one or more of these sources.

According to an embodiment, storage 260 of system 200 may store one or more algorithms, modules, and/or instructions to carry out one or more functions or steps of the methods described or otherwise envisioned herein. For example, the system may comprise, among other instructions or data, a trained diastolic function prediction algorithm 262, and/or reporting instructions 263.

According to an embodiment, the trained diastolic function prediction algorithm 262 is configured to analyze a received plurality of 2D echocardiographic images of the patient's heart, or a selected subset of the plurality of 2D echocardiographic images of the patient's heart, to estimate the left ventricular end-diastolic pressure (LVEDP) of the patient's heart. According to one embodiment, the trained diastolic function prediction algorithm may also analyze received clinical information about the patient, but this is not essential. Other input to the trained diastolic function prediction algorithm is possible. According to an embodiment, the trained diastolic function prediction algorithm estimates the LVEDP of the patient's heart using a wide variety of different classifier and/or machine learning algorithms as described or otherwise envisioned herein. According to an embodiment, the trained diastolic function prediction algorithm of the diastolic function analysis system can be trained according to a wide variety of methods and approaches. As one example, the algorithm may comprise a neural network approach. The trained diastolic function prediction algorithm 262 is trained using a training data set as described or otherwise envisioned herein.

According to an embodiment, reporting instructions 263 direct the system to generate and provide to a user via a user interface information comprising the classification of the patient's diastolic function - which may be normal, indeterminate, abnormal, or other possible classifications - as well as optionally providing the estimated LVEDP determined by the diastolic function prediction algorithm. The provided information may include additional information such as information about the patient, including the optional demographic information provided to and/or used by the system, the patient's name, and/or a treatment or intervention recommendation, among many other types of information. The information may be communicated by wired and/or wireless communication via the user interface of the system or of another device. For example, the system may communicate the information to a mobile phone, computer, laptop, wearable device, and/or any other device configured to allow display and/or other communication of the report. The user interface can be any device or system that allows information to be conveyed and/or received, and may include a display, a mouse, and/or a keyboard for receiving user commands.

Accordingly, within the context of the disclosure herein, aspects of the embodiments may take the form of a computer program product embodied in one or more non-transitory computer-readable media having computer readable program code embodied thereon. Thus, according to one embodiment is a non-transitory computer-readable storage medium comprising computer program code instructions which, when executed by a processor, enables the processor to carry out a method including: (i) receiving a plurality of 2D echocardiographic images of the patient's heart; (ii) analyzing, by a trained diastolic function prediction algorithm, the plurality of 2D echocardiographic images of the patient's heart to estimate left ventricular end-diastolic pressure (LVEDP); (iii) classifying the patient's diastolic function as normal or abnormal based on the estimated LVEDP; and (iv) providing, to a user via a user interface, an indication of the patient's diastolic function as normal or abnormal. The program code may perform entirely on a user's computer, partly on a user's computer, as a stand-alone software package, partly on a user's computer and partly on a remote computer, or entirely on a remote computer or server.

According to an embodiment, the diastolic function analysis system is configured to process many thousands or millions of datapoints in the input data used to train the system, as well as to process and analyze the received plurality of 2D echocardiographic images. For example, generating a functional and skilled trained system using an automated process such as feature identification and extraction and subsequent training requires processing of millions of datapoints from input data and the generated features. This can require millions or billions of calculations to generate a novel trained system from those millions of datapoints and millions or billions of calculations. As a result, the trained system is novel and distinct based on the input data and parameters of the machine learning algorithm, and thus improves the functioning of the diastolic function analysis system. Thus, generating a functional and skilled trained system comprises a process with a volume of calculation and analysis that a human brain cannot accomplish in a lifetime, or multiple lifetimes. By providing an improved patient analysis, this novel diastolic function analysis system has an enormous positive effect on patient diagnosis and care compared to prior art systems.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A method (100) for classifying a patient's diastolic function, comprising:
receiving (120), from an ultrasound device (280), a plurality of 2D echocardiographic images of the patient's heart;
analyzing (150), by a trained diastolic function prediction algorithm, the plurality of 2D echocardiographic images of the patient's heart to estimate left ventricular end-diastolic pressure (LVEDP);
classifying (160) the patient's diastolic function as normal or abnormal based on the estimated LVEDP; and
providing (170), to a user via a user interface, an indication of the patient's diastolic function as normal or abnormal.

2. The method of claim 1, further comprising the step of selecting (130), by a trained image selection algorithm, a subset of the plurality of received 2D echocardiographic images of the patient's heart for analysis, wherein the image selection algorithm is trained to select 2D echocardiographic images as being optimal for analysis, wherein said analyzing step comprises analyzing the selected subset of the plurality of received 2D echocardiographic images of the patient's heart.

3. The method of claim 1 or 2, further comprising the step of receiving (140) clinical information about the subject, wherein the trained diastolic dysfunction prediction algorithm also analyzes the received clinical information to classify the patient's diastolic function as normal or abnormal.

4. The method of any of claims 1-3, further comprising the step of receiving (122), via a user interface, input from a user to initiate an analysis by the trained diastolic dysfunction prediction algorithm.

5. The method any of of claims 1-4, wherein the patient's diastolic function is classified as normal when the estimated LVEDP is equal to or less than 10 mmHg.

6. The method of any of claims 1-5, wherein the patient's diastolic function is classified as abnormal when the estimated LVEDP is equal to or greater than 15 mmHg.

7. The method of any of claims 1-6, wherein the trained diastolic dysfunction prediction algorithm is further configured to classify the patient's diastolic function as indeterminate when the estimated LVEDP is between 10 mmHg and 15 mmHg.

8. A system (200) for classifying a patient's diastolic function, comprising:
an ultrasound device (280) configured to obtain a plurality of 2D echocardiographic images of the patient's heart;
a trained diastolic function prediction algorithm (262) trained to estimate left ventricular end-diastolic pressure (LVEDP) from the plurality of 2D echocardiographic images of the patient's heart;
a user interface (240);
a processor (220) configured to: (i) analyze, using the trained diastolic function prediction algorithm, the plurality of 2D echocardiographic images of the patient's heart to estimate LVEDP; (ii) classify the patient's diastolic function as normal or abnormal based on the estimated LVEDP; and (iii) direct the user interface to provide an indication of the patient's diastolic function as normal or abnormal.

9. The system of claim 8, wherein the processor is further configured to select, using a trained image selection algorithm, a subset of the plurality of received 2D echocardiographic images of the patient's heart for analysis, wherein the image selection algorithm is trained to select 2D echocardiographic images as being optimal for analysis, wherein said analyzing step comprises analyzing the selected subset of the plurality of received 2D echocardiographic images of the patient's heart.

10. The system of claim 8 or 9, wherein the processor is further configured to receive clinical information about the subject, wherein the trained diastolic function prediction algorithm also analyzes the received clinical information to classify the patient's diastolic function as normal or abnormal.

11. The system of any of claims 8-10, wherein the processor is further configured to receive, via the user interface, input from a user to initiate an analysis by the trained diastolic dysfunction prediction algorithm.

12. The system of any of claims 8-11, wherein the patient's diastolic function is classified as normal when the estimated LVEDP is equal to or less than 10 mmHg.

13. The system of any of claims 8-12, wherein the patient's diastolic function is classified as abnormal when the estimated LVEDP is equal to or greater than 15 mmHg.

14. The system of any of claims 8-13, wherein the trained diastolic function prediction algorithm is further configured to classify the patient's diastolic function as indeterminate when the estimated LVEDP is between 10 mmHg and 15 mmHg.

15. A non-transitory computer-readable storage medium comprising computer program code instructions which, when executed by a processor, enables the processor to carry out the method according to any of claims 1-7.
